# EUROPÄISCHE PATENTANMELDUNG

(11) **EP 2 149 334 A1**
(43) Veröffentlichungstag der Anmeldung: **03.02.2010**
(21) Anmeldenummer: 09163983.1
(22) Anmeldetag: 29.06.2009
(51) Int. Cl.: A61B 5/0215

(54) **Intravaskuläre Messung strömungsmechanischer Parameter mittels OFW-Transponder**

(30) Priorität: 28.07.2008 DE 102008040790
(71) Anmelder: Biotronik VI Patent AG, 6341 Baar (CH)
(72) Erfinder: Tittelbach, Michael, 90429, Nürnberg (DE); Harder, Claus, 91080, Uttenreuth (DE)
(74) Vertreter: Lindner-Vogt, Karin L.

(57) **Zusammenfassung**

Ein implantierbarer Sensor zur Messung strömungsmechanischer Parameter mit einem Oberflächenwellen-Transponder zur Erfassung vasomotorischer Größen und jeweils einem an zwei gegenüberliegenden Seiten des Oberflächenwellen-Transponders befestigten Haltestent.

## Beschreibung

Die Erfindung betrifft einen implantierbaren Sensor zur Erfassung strömungsmechanischer Kenngrößen des Blutflusses.

### Technologischer Hintergrund und Stand der Technik

Im Stand der Technik sind implantierbare Drucksensoren bekannt, welche in einem Blutgefäß platziert werden können und eine Bestimmung des Blutdrucks erlauben. Die Messergebnisse solcher Drucksensoren werden zur Überwachung der Herzleistung von Patienten mit einer Herzinsuffizienz oder ähnlichen Leiden eingesetzt. Ein solcher Drucksensor ist beispielsweise in US 2002/0045921 offenbart.

Um die von dem implantierbaren Drucksensor bestimmten Werte auszulesen, werden oft Telemetrieeinheiten benutzt, welche beispielsweise nach Aktivieren eines im Drucksensor befindlichen oder mit diesem verbundenen Magnetschalters durch einen Lesekopf die Messdaten über Funk übertragen. Da eine solche Telemetrieeinheit eine Spannungsversorgung per Batterie oder Induktion voraussetzt, werden solche Drucksensoren bevorzugt zusammen mit implantierbaren Herzschrittmachern eingesetzt, welche zunehmend über Telemetriefunktionen verfügen, welche es einem behandelnden Arzt erlauben, eine Vielzahl von medizinischen und betriebsbezogenen technischen Daten auszulesen.

Derartige Sensoren haben jedoch den Nachteil, dass sie den Blutfluss behindern und dabei neben der verschlechterten Durchblutung eine erhebliche Thrombosegefahr bedeuten, weil sich an dem den Blutfluss behindernden Drucksensor Zellen oder Blutplättchen und andere feste Bestandteile des Blutes anlagern können. Wird eine solche Ablagerung vom Blutfluss vom Drucksensor abgerissen, kann es zu einem Verschluss eines kleineren Blutgefäßes, in das der abgerissene Klumpen transportiert wird, und dadurch zu lebensgefährlichen Gesundheitsbeeinträchtigungen kommen. Dieser Gefahr wird daher durch Antikoagulation, also durch Gabe von gerinnungshemmenden Medikamenten, begegnet, was jedoch entsprechende Risiken und Nebenwirkungen nach sich zieht.
Ferner haben unmittelbar im Blutfluß befindliche Drucksensoren den Nachteil, dass die oben beschriebenen Anlagerungen zu einer erheblichen technischen Beeinträchtigung des Sensors - bis hin zu einem Totalausfall - führen können.
Es besteht daher ein Bedarf für einen implantierbaren Sensor, der einen oder mehrere der obengenannten Mängel des Standes der Technik überwindet.

### Zusammenfassung der Erfindung

Die Erfindung gibt daher einen implantierbaren Sensor zur Messung strömungsmechanischer Parameter an, der über einen Oberflächenwellen-Transponder (OFW-Transponder) zur Erfassung vasomotorischer Größen und jeweils einen an zwei gegenüberliegenden Seiten des OFW-Transponders befestigten Haltestent verfügt. Der Begriff "Haltestent" bedeutet hier ein einem Stent ähnliches Halteelement, welches jedoch nicht die bei einem gewöhnlichen Stent gewünschte Radialfestigkeit zu besitzen braucht, sondern lediglich dazu dient, den OFW-Transponder im Blutgefäß an dessen Wand zu fixieren. Insbesondere können die Haltestents auch kürzer als gewöhnliche Stents sein und eine deutlich geringere Radialfestigkeit aufweisen.

Die Erfindung schließt den Gedanken mit ein, dass eine Bestimmung von Blutdruck und Blutfluss auch über die Vasomotorik des Blutgefäßes als Surrogatparameter möglich ist. Dabei macht die Erfindung sich die Tatsache zunutze, dass sich ein Blutgefäß mit variierendem Blutdruck unterschiedlich weitet. Weil der Blutdruck infolge eines Herzschlages kurzzeitig ansteigt, um dann wieder abzuklingen, wird das Blutgefäß fortlaufend periodisch verformt. Die Erfindung beruht daher auf der Erkenntnis, dass die Stärke der Verformungen der Blutgefäße einen Rückschluss auf den herrschenden Blutdruck erlaubt, so dass sich ein verbesserter Blutdrucksensor realisieren lässt, indem der Blutdruck indirekt über die Verformung eines Blutgefäßes bestimmt wird. Demzufolge ist der OFW-Transponder bevorzugt als Biegebalken ausgebildet.

Der OFW-Transponder erlaubt gleichzeitig ein Bestimmen der genannten vasomotorischen Größe sowie deren Auslesen, ohne dass eine Telemetrieeinheit und somit eine Spannungsversorgung per Batterie oder Induktion notwendig wäre. Aus diesem Grund kann der implantierbare Sensor unabhängig von einem elektromedizinischen Implantat wie einem Herzschrittmacher eingesetzt werden und besitzt kleine Abmessungen, welche eine Behinderung des Blutflusses vermeiden helfen. Die Verwendung des OFW-Transponders als Sensor in einem Blutgefäß besitzt zudem den weiteren Vorteil, dass ein Bewuchs des Sensors mit Zellen die Messqualität nicht beeinträchtigt. Die Blutdruckmessung kann durch eine Vergleichsmessung mit herkömmlichen Mitteln kalibriert werden. Diese Vergleichsmessung sollte in regelmäßigen Abständen wiederholt werden, weil sich die Messbedingungen durch das Einwachsen des Sensors verändern können.

Aufgrund der beiden an den Enden des OFW-Transponders befestigten Haltestents wird der OFW-Transponder gegen die Wand des Blutgefäßes gedrückt, weshalb der Blutfluss durch das Blutgefäß nur geringfügig behindert wird, da das Lumen des Blutgefäßes im wesentlichen frei bleibt. Zudem wächst der gesamte OFW-Transponder mit der Zeit in die Gefäßwand ein, wodurch eine Thrombosegefahr praktisch nicht mehr gegeben ist, weshalb auch die oben genannte langfristige Belastung durch gerinnungshemmende Medikamente entfällt. Eine besonders bevorzugte Ausführung der Erfindung sieht zudem Haltestents vor, welche aus einem biokorrodierbaren Material, also einem Material, das im menschlichen Körper mit der Zeit abgebaut werden kann, gefertigt sind. Die Zeit, in der die Haltestents erwartungsgemäß abgebaut sein werden, sollte dabei so gewählt werden, dass ein vollständiges Einwachsen des OFW-Transponders in die Gefäßwand sichergestellt ist, bevor die Haltestents abgebaut sind bzw. nicht mehr vermögen, den OFW-Transponder an der Gefäßwand zu fixieren. Bei dieser Ausführung ist die Behinderung des Blutflusses durch den Blutdrucksensor nach Abbau der Haltestents minimiert.

Für die Haltestents wird der Einsatz von Magnesium oder Reineisen sowie biokorrodierbaren Basislegierungen der Elemente Magnesium, Eisen, Zink, Molybdän und Wolfram vorgeschlagen. Bevorzugt wird dabei eine biokorrodierbare Magnesiumlegierung verwendet. Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten.

Die Magnesiumlegierung ist so in ihrer Zusammensetzung zu wählen, dass sie biokorrodierbar ist. Als Prüfmedium zur Testung des Korrosionsverhaltens von Legierungen dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCI 0,4 g/l, MgSO4 0,1 g/l, NaHCO3 2,2 g/l, Na2HPO4 0,126 g/l, NaH2PO4 0,026 g/I). Eine Probe des zu untersuchenden Werkstoffs wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Das Auslesen des OFW-Transponders erfolgt mittels hochfrequenter Abfragepulse. Der Abfragepulse wird von einer Antenne des OFW-Transponders empfangen und durch die Kraftwirkung der empfangenen elektromagnetischen Wellen in eine mechanische Oberflächenwelle gewandelt. Die Oberflächenwelle breitet sich im OFW-Transponder aus, wobei sie sich mit einer um mehrere Größenordnungen geringeren Ausbreitungsgeschwindigkeit als die des Abfragepulses im Medium Luft ausbreitet. Dadurch sind bereits sämtliche Reflektionen des Abfragepulses an umstehenden Hindernissen abgeklungen, bevor die Oberflächenwelle nach Durchwandern des OFW-Transponders (ggf. nach Reflektion an dessen anderen Ende) aufgrund des piezoelektrischen Effekts wieder in eine elektromagnetische Welle gewandelt und per Antenne abgestrahlt wird. Das abgestrahlte Signal ist um mehrere Größenordnungen schwächer als der Abfragepuls, da jedoch zum Zeitpunkt der Antwort des OFW-Transponders die Echos des Abfragepulses bereits abgeklungen sind, kann die Antwort des OFW-Transponders mit geeigneten Empfängern empfangen werden.

Die Oberflächenwelle breitet sich im OFW-Transponder abhängig von Parametern wie der Temperatur und mechanischer Verformung aus und wird von diesen beeinflusst, weshalb aus der Antwort des OFW-Transponders auch auf diese Parameter rückgeschlossen werden kann. Da in einem Blutgefäß prinzipbedingt eine nahezu konstante Temperatur von rund 37 Grad Celsius herrscht, wird die Oberflächenwelle hauptsächlich durch die Verformung des OFW-Transponders aufgrund der Kraftwirkung des sich mit jedem Herzschlag dehnenden und wieder kontrahierenden Blutgefäßes beeinflusst, was sich insbesondere in einer Variation der Laufzeiten niederschlägt. Daher kann der Blutdruck über die Verzögerung der Antwort des OFW-Transponders gegenüber dem Abfragepulse abgelesen werden. Zusätzlich können auf dem OFW-Transponder Reflektoren aufgebracht werden, die eine Teilreflektion der Oberflächenwelle bewirken. Jede Teilreflektion bewirkt einen eigenen Antwortpuls des OFW-Transponders, wobei die zeitliche Lage der Antwortpulse relativ zueinander durch die räumliche Anordnung der Reflektoren auf dem OFW-Transponder bestimmt ist. Demzufolge kann auch die Veränderung des zeitlichen Abstandes zweier oder mehrerer Antwortpulse als Messwert herangezogen werden. Außerdem lassen sich zusätzliche Reflektoren auf den OFW-Transponder anbringen, wobei die räumliche Anordnung der Reflektoren eine charakteristische Antwortmarke bildet. Derlei ist aus dem Bereich der RFID-Transponder bekannt. Eine Ausführungsform der Erfindung mit solchen zusätzlichen Reflektoren besitzt den Vorteil, dass eine automatisierte Identifizierung des implantierten Blutdrucksensors und damit im Einsatz des jeweiligen Patienten möglich ist, wodurch beispielsweise automatisch Messdaten vergangener Blutdruckmessungen dieses Patienten zum Vergleich der aktuellen Messdaten herangezogen und ggf. auf einem Bildschirm visualisiert sowie die aktuellen Blutdruckdaten als dem Patienten zugeordnet gespeichert werden können. Auch erlaubt es die charakteristische Antwortmarke oder ein Teil derselben, das Antwortsignal signaltechnisch leichter von dem immer gegebenen Hintergrundrauschen zu separieren, wenn die Antwortmarke vor Durchführung der Messung bekannt ist. Ist ein Reflektor auf dem OFW-Transponder so positioniert, dass sich ein möglichst großer zeitlicher Abstand zwischen dem Zeitpunkt der Reflektion der hinlaufenden Oberflächenwelle am Reflektor und der rücklaufenden, am Ende des OFW-Transponders reflektierten Oberflächenwelle ergibt, kann die Bestimmung der Messgröße anhand der relativen Lage der beim Passieren des Reflektors und durch die reflektierte Oberflächenwelle erzeugten Antwortpulse erfolgen. Zu diesem Zweck verfügt eine bevorzugte Ausführungsform der Erfindung über einen oder mehrere Reflektoren, von denen wenigstens ein erster Reflektor auf einem Träger des Oberflächenwellen-Transponders mit einem ersten Abstand zu einem Paar von Interdigitalwandlern und mit einem zweiten Abstand zu einem dem Paar von Interdigitalwandlern gegenüberliegenden Ende des Oberflächenwellen-Transponders angeordnet ist, wobei der zweite Abstand größer ist als der erste Abstand. Der zweite Abstand ist dabei bevorzugt wenigstens doppelt so groß wie der erste Abstand, besser jedoch fünf- oder zehnmal größer als der erste Abstand.

Das Auslesen des OFW-Transponders kann bis zu tausende mal pro Sekunde erfolgen. Da der Herzschlag mit rund einem Schlag pro Sekunde erfolgt, können die über einen Zeitraum von beispielsweise 10 bis 250 ms gesammelten Messergebnisse zur Verbesserung der Messgenauigkeit gemittelt werden. Weil die Ausbreitungsgeschwindigkeit der Oberflächenwelle im Transponder zudem deutlich höher ist als die der durch den Herzschlag verursachten Druckwelle im Blutgefäß, kann der zeitliche Verlauf des Blutdrucks zwischen zwei Herzschlägen bestimmt werden, wodurch sich der systolische und der diastolische Blutdruck einfach aus der Transientenmessung ablesen lassen.

Um eine bessere Gewebeverträglichkeit zu gewährleisten, ist der Oberflächenwellen-Transponder vorzugsweise mit einer biokompatiblen Beschichtung versehen. Die biokompatible Beschichtung ist bevorzugt Polyurethan oder Parylene.

Die für das Empfangen des Abfragepulses und das Abstrahlen der Antwort benötigte(n) Antenne(n) ist bei einer Ausführungsvariante der Erfindung besonders vorteilhaft in den implantierbaren Sensor integriert, bei der wenigstens einer der Haltestents eine Antenne umfasst oder als solche fungiert. Die Antenne kann dabei vorteilhaft als Rahmenantenne ausgeführt sein, welche vom Haltestent bei der Expansion des Haltestents aufgespannt wird. Ebenso ist es möglich, den Haltestent oder die Haltestents durch elektrisch isolierende Bereiche in halb- oder viertelkreisförmige Schalen oder Abschnitte zu unterteilen, welche dann zwei Hälften einer Halbwellendipolantenne bilden. Auch können zwei Hälften einer Halbwellendipolantenne auf dem Haltestent aufgebracht sein, so dass die Halbwellendipolantenne von dem Haltestent bei der Expansion aufgespannt wird.

Bei einer Ausführungsform mit biokorrodierbaren Haltestents ist die Antenne aus einem nicht biokorrodierbaren Material hergestellt.

Alternativ oder zusätzlich kann der implantierbare Sensor eine Dipolantenne aufweisen, wobei eine Längserstreckungsrichtung der Dipolantenne entlang einer Verbindungslinie zwischen den Haltestents verläuft. Die Dipolantenne fügt sich hierbei räumlich vorteilhaft in den Sensor ein und ist wenigstens näherungsweise parallel zur Flussrichtung des Blutes ausgerichtet, so dass der Blutfluss nur minimal behindert wird.

Vorzugsweise sind die Haltestents als selbstexpandierende Stents ausgeführt. Alternativ können die Haltestents auch als ballonexpandierte Stents ausgeführt sein.

Der implantierbare Sensor kann mit einem Marker, der die leichte Ortung mittels Röntgen oder MRT erlaubt, versehen sein.

Die Erfindung wird im Folgenden anhand zweier Abbildungen näher erläutert. Es zeigen
- Fig. 1: ein Prinzipschaltbild eines Oberflächenwellen-Transponders und
- Fig. 2: einen implantierbaren Sensor gemäß der Lehre der Erfindung.

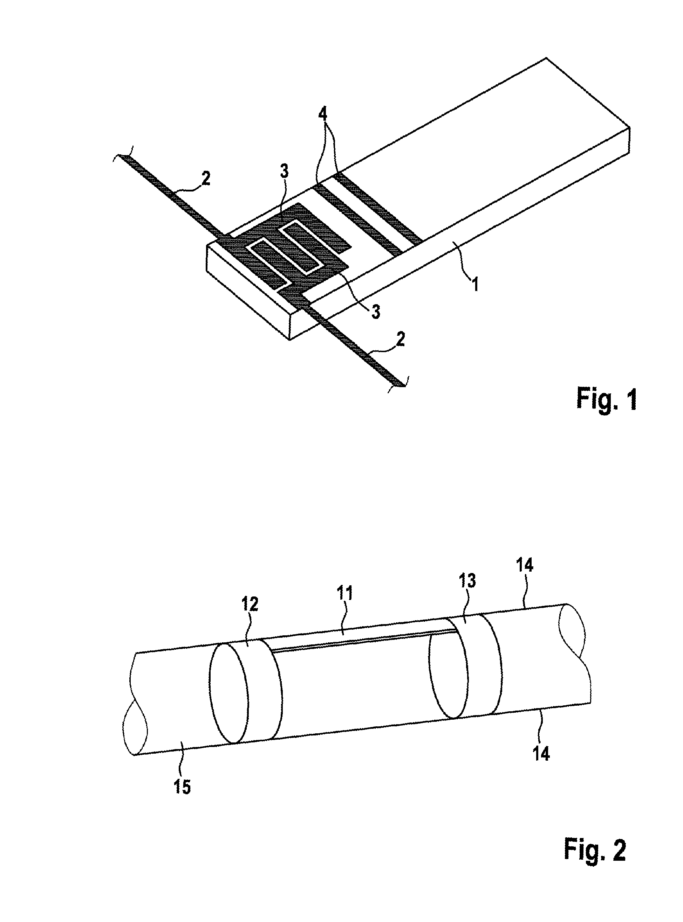

Fig. 1 zeigt ein Prinzipschaltbild eines Oberflächenwellen-Transponders. Auf einem Träger 1, der aus einem piezoelektrischen Einkristall besteht, sind an einem Ende des Trägers 1 mit einer Antenne 2 verbundene Interdigitalwandler 3 aufgebracht, welche eine verzahnte Struktur aufweisen und aufgrund der bei Empfang eines Abfragepulses über die Antenne 2 auftretenden Kraftwirkung des elektromagnetischen Feldes auf das piezoelektrische Material des Trägers 1 eine Oberflächenwelle im Träger 1 erzeugen. Die Antenne 2 ist dabei als geteilter Halbwellendipol ausgeführt, so dass die Interdigitalwandler 3 in der Mitte der Antenne 2 das Empfangssignal abgreifen. Die Oberflächenwelle durchläuft den Träger 1 und ist dort Beeinflussungen aufgrund von Änderungen der Weglänge durch Verformung des Trägers 1 und von elastischen Kristallkonstanten ausgesetzt. Auf dem Träger 1 sind zwei Reflektoren 4 aufgebracht, welche aufgrund des festgelegten Abstandes zueinander Reflektionen der Oberflächenwelle mit einem bestimmten zeitlichen Abstand zueinander erzeugen. Im abgebildeten Beispiel sind zwei Reflektoren 4 dicht nebeneinander und nah an den Interdigitalwandlern 3 angeordnet, so dass die Oberflächenwelle die Reflektoren 4 jeweils einmal vor und nach der Reflektion am den Interdigitalwandlern 3 gegenüberliegenden Ende des Trägers 1 passiert. Aufgrund der Reflektion an den Reflektoren 4 wird eine charakteristische Marke von zwei zeitlich dicht aufeinander folgenden Antwortpulsen erzeugt, welche sich auch bei Hintergrundrauschen leicht detektieren lässt. Da die beiden Antwortpulse so dicht aufeinander folgen, dass nur eine minimale Beeinflussung des zeitlichen Abstandes der Antwortpulse durch die Messgröße erfolgt, ist dieser nahezu konstant. Wird nun die Ausbreitungsgeschwindigkeit der Oberflächenwelle durch den Träger 1 aufgrund von Verformung des Trägers 1 durch das Blutgefäß verändert, ergibt sich eine entsprechende Beeinflussung der Oberflächenwelle, welche sich in der Ausbreitungsgeschwindigkeit der Oberflächenwelle und somit im zeitlichen Abstand des Paares von Antwortpulsen zum durch die zurückkehrende reflektierte Oberfläche verursachten Antwortpuls niederschlägt. Nachdem die Oberflächenwelle am Ende des Trägers 1 reflektiert wurde und zum Interdigitalwandler 3 zurückgewandert ist, wandelt der Interdigitalwandler 3 die akustische Oberflächenwelle in ein elektromagnetisches Signal zurück, welches über die Antenne 2 abgestrahlt wird. Da dieses abgestrahlte Signal einen Rückschluss auf die Bedingungen im Träger 1 während der Ausbreitung der Oberflächenwelle zulässt, fungiert der Oberflächenwellen-Transponder als Sensor und erlaubt das Erfassen strömungsmechanischer Messgrößen, welche als Surrogatparameter für den Blutdruck und Blutfluss dienen können. Die Reflektoren 4 sind optional, da im Abfragegerät der Zeitpunkt des Aussendens des Abfragepulses bekannt ist und somit der zeitliche Abstand zu einem einzigen Antwortpuls, welcher sich nach zweimaligem Durchlaufen des Trägers 1 ergibt, bestimmt werden kann. Allerdings hat die in Fig. 1 gezeigte Ausführungsform mit Reflektoren 4 den Vorteil, dass die mit dem Abstand zwischen dem Abfragegerät und der Position des OFW-Transponders variierende Laufzeit von Abfrage- und Antwortpuls aus der Messung eliminiert wird, was die Messgenauigkeit erhöht.

Es ist auch möglich, zwei Paare Antennen 2 und Interdigitalwandler 3 vorzusehen, welche an den beiden gegenüberliegenden Enden des Trägers 1 angeordnet sind. In diesem Fall wird die Oberflächenwelle nach nur einmaligem Durchlaufen des Trägers 1 wieder in ein elektromagnetisches Signal gewandelt und abgestrahlt. Diese Ausführungsform besitzt jedoch den Nachteil, dass die Oberflächenwelle nur einmal der Beeinflussung durch die Messgröße im Träger 1 unterworfen und somit weniger stark beeinflusst wird. Zudem erfolgt die Antwort des Transponders in der halben Zeit, da die Wegstrecke im Transponder nicht wie in der Ausführungsform mit nur einer Antenne 2 verdoppelt ist.

Fig. 2 zeigt einen implantierbaren Sensor gemäß der Lehre der Erfindung. Ein Oberflächenwellen-Transponder 11 ist an seinen beiden Schmalseiten mit jeweils einem Haltestent 12, 13 verbunden. Die Haltestents 12, 13 sind in expandiertem Zustand gezeigt, in welchem sie vollumfänglich auf der Gefäßwand 14 aufliegen und somit den implantierbaren Sensor im Blutgefäß verankern. Da die Haltestents 12, 13 ringförmig ausgeführt sind, bleibt das Gefäßlumen 15 frei, so dass der Blutfluss nur minimal beeinträchtigt wird. Auf dem implantierbaren Sensor bildet sich mit der Zeit eine als Neointima bezeichnete Schicht, die luminal von einer monozellulären Endothelschicht begrenzt wird, so dass der OFW-Transponder mit der Zeit vollständig in die Gefäßwand einwächst.

## Patentansprüche

1. Implantierbarer Sensor zur Messung strömungsmechanischer Parameter mit einem Oberflächenwellen-Transponder zur Erfassung vasomotorischer Größen und jeweils einem an zwei gegenüberliegenden Seiten des Oberflächenwellen-Transponders befestigten Haltestent.

2. Der implantierbare Sensor aus Anspruch 1, bei dem der Oberflächenwellen-Transponder als Biegebalken ausgebildet ist.

3. Der implantierbare Sensor aus einem der vorhergehenden Ansprüche, bei dem der Oberflächenwellen-Transponder mit einer biokompatiblen Beschichtung versehen ist.

4. Der implantierbare Sensor aus Anspruch 3, bei dem die biokompatible Beschichtung Polyurethan ist.

5. Der implantierbare Sensor aus einem der vorhergehenden Ansprüche, bei dem wenigstens einer der Haltestents eine Antenne umfasst oder ausgebildet ist, als Antenne zu fungieren.

6. Der implantierbare Sensor aus Anspruch 5, bei dem die Antenne als Rahmenantenne ausgeführt ist und bei dem der wenigstens eine Haltestent ausgebildet ist, die Rahmenantenne beim Expandieren des wenigstens einen Haltestents aufzuspannen.

7. Der implantierbare Sensor aus Anspruch 5, bei dem der wenigstens eine Haltestent zwei elektrisch voneinander isolierte Abschnitte aufweist, welche ausgebildet sind, als Hälften einer Halbwellendipolantenne zu fungieren.

8. Der implantierbare Sensor aus einem der vorhergehenden Ansprüche, bei dem die Haltestents als selbstexpandierende Stents ausgeführt sind.

9. Der implantierbare Sensor aus einem der Ansprüche 1 bis 8, bei dem die Haltestents als ballonexpandierte Stents ausgeführt sind.

10. Der implantierbare Sensor aus einem der vorhergehenden Ansprüche, bei dem die Haltestents wenigstens teilweise aus einem biokorrodierbaren Material bestehen.

11. Der implantierbare Sensor aus Anspruch 10, bei dem das biokorrodierbare Material eine Magnesiumlegierung ist.

12. Der implantierbare Sensor aus einem der vorhergehenden Ansprüche, der über einen Marker verfügt.

13. Der implantierbare Sensor aus einem der vorhergehenden Ansprüche, bei dem der Oberflächenwellen-Transponder über einen oder mehrere Reflektoren verfügt.

14. Der implantierbare Sensor aus Anspruch 13, bei dem wenigstens ein erster Reflektor auf einem Träger des Oberflächenwellen-Transponders mit einem ersten Abstand zu einem Paar von Interdigitalwandlern und mit einem zweiten Abstand zu einem dem Paar von Interdigitalwandlern gegenüberliegenden Ende des Oberflächenwellen-Transponders angeordnet ist, wobei der zweite Abstand größer ist als der erste Abstand.

15. Der implantierbare Sensor aus Anspruch 14, bei dem der zweite Abstand wenigstens zweimal, fünfmal oder zehnmal größer ist als der erste Abstand.
